# EUROPEAN PATENT SPECIFICATION

(11) **EP 1 701 760 B1**
(45) Date of publication and mention of the grant of the patent: **22.02.2012**
(21) Application number: 05705331.6
(22) Date of filing: 07.01.2005
(51) Int. Cl.: A61M 25/00, A61M 25/10

(54) **MULTILUMEN CATHETERS**
MEHRLUMIGE KATHETER
CATHETERS A LUMIERES MULTIPLES

(30) Priority: 09.01.2004 US 535618 P
(43) Date of publication of application: 20.09.2006
(60) Divisional application: 10185829.8
(73) Proprietor: Corazon Technologies, Inc., Menlo Park, CA 94025 (US)
(72) Inventor: CONSTANTZ, Brent R., Cupertino, California 95014 (US)
(74) Representative: Duxbury, Stephen
(86) International application number: PCT/US2005/000617
(87) International publication number: WO 2005/070110

(56) References cited:
- WO-A2-03/080151
- US-A- 4 911 163
- US-A- 5 149 330
- US-A1- 2001 039 411
- US-A1- 2002 026 145
- US-A1- 2004 082 910

## Description

### Field of the Invention

The field of this invention is atherosclerosis and related vascular conditions, and particularly catheter devices used for treating such conditions.

### Background of the Invention

The formation of plaques or lesions (atherosclerotic plaques or lesions), on vascular tissue, such as the inner surface of blood vessels, aortic valves, etc., is a major component of various vascular disease conditions For example, plaques on heart related vascular structures, e.g., coronary artery intima, heart valves, etc., are often implicated in various heart disease conditions. Likewise, plaques or lesions present on the intima of peripheral vessels, e.g., arteries, are often implicated in various peripheral vascular disease conditions.

A variety of different protocols have been developed for treating diseases associated with the presence of vascular lesions or plaques. Such treatment methodologies generally involve mechanical removal or reduction of the lesion, and include: bypass surgery, balloon angioplasty, mechanical debridement, atherectomy, valve replacement, and the like. Despite the plethora of different treatment strategies that have been developed for the treatment of such vascular disease conditions, there are disadvantages associated with each technique, such as tissue damage, invasiveness, etc. For example, restenosis is a common complication that results in arteries in which lesions have been mechanically removed.

As such, there is continued interest in the development of new treatment protocols for the removal of vascular lesions from vascular tissue, as well as catheter devices that are used in such protocols.
A multilumen catheter system is known from WO 03/080151

### Literature

U.S. Patents of interest include: 4,329,994; 4,838,881; 5,149,330; 5,167,623; 5,207,6485; 542,937;6,004,310; and 6,013,068. Also of interest are U.S. Patent Nos.: 4,445,892; 4,573,966; 4,610,662; 4,636,195; 4,655,746; 4,824,436; 4,911,163; 4,976,733; 5;059;178; 5,090,960; 5,167,628; 5,195,955; 5,222,941; 5,380,284; 5,443,446; and 5,462,529. See also: WO 00/03651; WO 01/13985; WO 01/15767; WO 01/39783; WO 01/70320; and WO 02/15958 .

### SUMMARY OF THE INVENTION

according to the appended claims, provided are systems for use in flushing a vascular site with fluid, usually at least two different fluids, where the subject systems are made up of a delivery catheter inside of an aspiration catheter. Multilumen aspiration and delivery catheters, are provided. The subject multilumen catheters include a proximal end and a distal end separated by a non-coaxial multilumen tube. In addition, kits comprising various components of the subject systems, e.g:, at least two different multilumen catheters, are provided. The subject multilumen aspiration and delivery catheters, systems and kits find use in a variety of different applications in which it is desired to flush a vascular site with at least one and preferably two different fluids, where particular applications of interest include the treatment of vascular lesions, including coronary artery vascular lesions.

### BRIEF DESCRIPTION OF THE FIGURES

Each of the following figures provides examples diagrammatically illustrating aspects of the present invention.

Figs. 1A and 1B each provide a representation of an aspiration catheter according to the subject invention.

Figs. 2A and 2B each provide a representation of a total occlusion catheter according to the subject intention.

Figs. 3A and 3B each provide a representation of a partial occlusion catheter according to the subject invention.

Fig. 4 provides a representation of a total occlusion catheter system according to the subject invention.

Fig. 5 provides a representation of a partial occlusion catheter system according to the subject invention.

### DESCRIPTION OF THE SPECIFIC EMBODIMENTS

according to the appended claims, provided are systems for use in flushing a vascular site with fluid, usually at least two different fluids, where the subject systems are made up of a delivery catheter inside of an aspiration catheter. Multilumen aspiration and delivery catheters, are provided. The subject multilumen catheter include a proximal end and a distal end separated by a non-coaxial multilumen tube. In addition, kits comprising various components of the subject systems, e.g., at least two different multilumen catheters, are provided. The subject multilumen aspiration and delivery catheters, systems and kits find use in a variety of different applications in which it is desired to flush a vascular site with at least one and preferably two different fluids, where particular applications of interest include the treatment of vascular lesions.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically exclude limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

In further describing the subject invention, the subject multilumen catheters are described first, both generally and in terms of the figures, followed by a description of the subject systems, kits and representative methods in which the catheters, systems and kits find use.

### MULTILUMEN CATHETERS

As summarized above, the present invention provides multilumen catheters. Specifically, the subject invention includes three different multilumen catheters, which can be used together as a system to simultaneously flush a vascular site with two distinct fluids. A common feature of each catheter of the subject invention is that they include a proximal end and a distal end separated by an elongated non-coaxial multilumen tube. By non-coaxial multilumen tube, it is meant a tube that includes at least two lumens that are non-coaxial, i.e., they do not share a common axis. The number of lumens in the multilumen tube may vary, but generally is 2 to 5, and is often 2 to 4, where in certain embodiments the number is 2 or 3. By elongated, it is meant that the distance between the proximal and distal ends is sufficient for the catheter to be inserted or introduced into the vascular system of a patient at a site remote from the vascular lesion that is to be treated through action of the distal end of the catheter, as is known in the art. Catheters intended for intravascular introduction will typically have a length in the range from 50 cm to 200 cm and an outer diameter in the range from 1 French (0.33 mm; Fr.) to 12 Fr., usually from 3 Fr. to 9 Fr. In the case of coronary catheters, the length is typically in the range from 125 to 200 cm, the diameter is preferably below 8 Fr., more preferably below 7 Fr., and most preferably in the range from 2 Fr. to 7 Fr. In certain embodiments, the elongated tubular element has a length of from about 80 to 200 cm, usually from about 90 to 180 cm and more usually from about 90 to 140 cm.

All of the subject multilumen catheters are further characterized in that they include a multiport manifold at their proximal ends. By multiport manifold is meant a manifold that includes two or more ports (in addition to the attachment structure of the mannifold to the proximal end of the elongated tube of the catheter), where the number of ports in the manifold may range from 2 to 4, depending on the particular multilumen catheter. In addition, at least one of the ports of the multiport manifold preferably has an attachment element for establishing a sealed fluid communication with the lumen of an external tubular element or analogous fluid conveying means, where this attachment structure is generally referred to herein as a luer-type connector or a luer-valve, where many different luer-type connectors or analogous attachment elements are known to those of skill in the art. In many embodiments, a luer valve is present at one or more ports of the multiport manifold, where the valve may be a male or female luer valve. Luer valves are disclosed in U.S. Patent Nos. 6,063,062; 6,039,302; 5,954,313; 5,947,954; 5,788,215; 5,775,671; 5,738,144; 5,549,651; 5,535,785; 5,474,544; 5,441,487; 5,372,143; 5,284,475 .

Another common feature of all of the multilumen catheters of the subject invention is that the elongated multilumen tube is typically a polymeric extruded element, which is made up of one or more biocompatible polymers that have been extruded to produce the non-coaxial multilumen tube. Biocompatible polymers of interest include, but are not limited to: polyimide, polyamide, PBAX™, polyethylene, polyisoprene, nylon and the like.

Although each of the disparate multilumen catheters share the above common features, there are differences between the different specific multilumen catheter designs of the subject invention. As such, each specific type of multilumen catheter of the subject invention, e.g., aspiration, total and partial multilumen catheters, will now be described separately in greater detail.

### Aspiration Catheter

The multilumen aspiration catheter of the subject invention is identified herein as the aspiration catheter, as it includes the lumen employed for aspiration when the catheter is used in a system for flushing a vascular site with fluid. The aspiration catheter is characterized by having a proximal end and a distal end separated by a non-coaxial three-lumen tube, where a four-port manifold is present at the proximal end. In the four-port manifold, at least three of the ports typically have luer type connectors, e.g., luer valves, while the fourth port has a valve capable of opening and closing around a tubular element, e.g., a delivery catheter, dilator, guidewire, etc., to produce a sealed relationship with the tubular element. In many embodiments, this sealing element is a Touhy-Borst valve or analogous structure, where Touhy-Borst valves are known to those of skill in the art and described in U.S. Pat. Nos.: 5,795,307 and 5,320,613 .

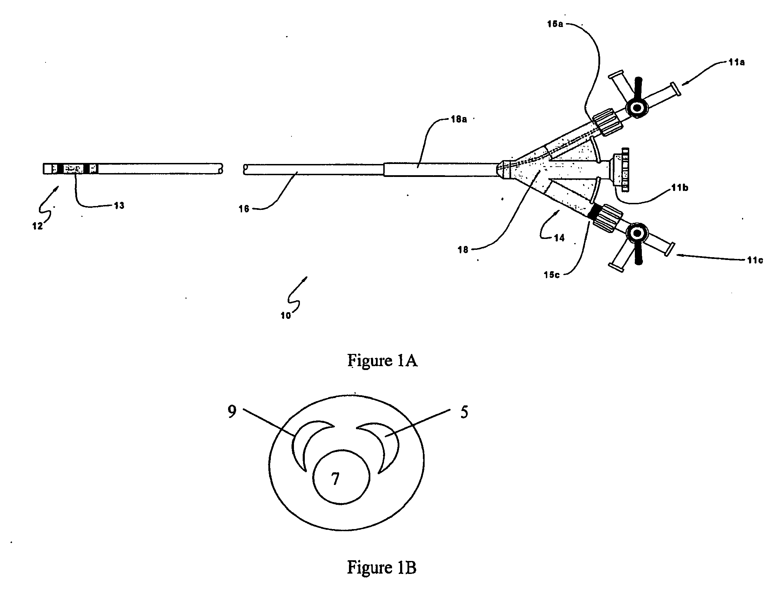

Figs. 1A and B provide a depiction of a representative aspiration catheter according to the subject invention. In Fig. 1A, aspiration catheter 10 has proximal end 14 and distal end 12 separated by elongated non-coaxial three-lumen tube 16. The length of elongated tube 16 should be long enough to provide for access of the distal end to the target vascular site upon introduction into the host, subject or patient at a remote site, and typically ranges from about 80 to 200 cm, usually from about 90 to 180 cm and more usually from about 90 to 140 cm. The outer diameter of the elongate tubular member 16 may vary depending on the target vascular site for which it is designed to be used. In other words, the outer diameter of the aspiration catheter is selected so as to provide for access of the distal end of the catheter to the vascular site via the vascular system from the remote point of entry, where the outer diameter typically ranges from about 1.0 to about 12.0 Fr., usually from about 1.0 to about 9.0 Fr. and more usually from about 1.0 to about 8.0 Fr. In the case of coronary catheters, the length is typically in the range from 125 to 200 cm, the diameter is typically below about 8.0 Fr., often below about 7.0 Fr., and typically in the range from about 2.0 Fr. to about 7.0 Fr.

The aspiration catheter is further characterized in that one of the three lumens, generally the larger of the three lumens, opens at the distal end, thereby providing a fluid entry site for fluid from the target vascular site to flow into the lumen. In Fig. 1B, this larger lumen is designated 7. The inner diameter of this lumen at the open distal end and along the entire length of the tube 16 is sufficient to house either a partial or total occlusion multilumen catheter, as described in greater detail below, and remove fluid from the vascular site at the desired rate, e.g., a rate that provides for substantially isometric or isobaric pressure in the vascular site during treatment, through the resultant annular space. This larger lumen 7 is also known as the aspiration lumen. The inner diameter of the aspiration lumen 7, at least at its distal end and generally along the entire length of the aspiration catheter, is sufficient to provide for adequate flow into the lumen of the catheter, and sometimes ranges from about 0.20 to 2.0, usually from about 0.25 to 1.75 and more usually from about 0.35 to 1.5 mm.

Also present at the distale end of the aspiration catheter 10 is a vessel occlusion means 13, where the vessel occlusion means is usually an inflatable balloon. The balloon 13 is one that is inflatable to a volume sufficient to substantially occlude the vessel in which the aspiration catheter is positioned, e.g., by pressing against the intimal surface of the vessel in which the aspiration catheter is positioned. The inflated balloon diameter generally ranges from about 2 to 30 mm, usually from about 5 to 20 mm. The inflated balloon length typically ranges from about 10 to 30 mm, usually from about 15 to 20 mm. The balloon is in fluid or gaseous communication with an inflation lumen 9 that is a second lumen of the three-lumen tube and runs the length of the aspiration catheter from the balloon to the proximal end, but does not open up at the distal end of the catheter. The inflation lumen typically has an inner diameter that ranges from about 0.1 to about 0.5 mm, usually from about 0.2 to about 0.4 mm. In certain embodiments, the aspiration catheter further includes a separate guidewire lumen (not shown). However, in many embodiments, a separate guidewire lumen is not present.

The third lumen of the three-lumen tube is typically employed for delivery of a dissolution fluid attenuating fluid to the target vascular site, e.g., for delivery of a buffer solution to the target vascular site. The cross-sectional area of this delivery lumen 5 typically ranges from about 0.03 to about 0.8 mm², usually from about 0.03 to about 0.4 mm² and more usually from about 0.07 to about 0.2 mm².

While the configuration of the representative three-lumen aspiration catheter depicted in Figures 1A and B shows circular and crescent shaped lumens, other configurations are also possible, e.g., two circular lumens, to semicircular lumens, etc.

At the proximal end 14 of aspiration catheter 10 is four-port manifold 18 (note that one of the ports is not shown in the figure). Four-port manifold 18 includes side or offset ports 11 a, 11 c and 11 d (not shown), as well as central port 11b. One of the offset ports, e.g., 11a is in fluid communication with the smaller lumen 9 and is attached to a balloon inflation means, e.g., a syringe filled with gas or fluid, during use. The second offset port, e.g., 11c, is in fluid communication the larger aspiration lumen 7 and is attached to a source of negative pressure, e.g., a vacuum, during use. The third offset port, 11d, is in fluid communication with a source or reservoir of dissolution fluid attenuating fluid. Offset ports 11 a, 11 c and 11 d (not shown) are further characterized in having or being in communication with luer valves 15a, 15c and 15d (not shown), respectively, for making connections with fluid conveyance means. In many embodiments, luer valves 15a, 15c and 15d (not shown) are female luer valves. Central port 11b is in fluid communication with the aspiration lumen 7 and provides the point of access for the total and partial occlusion catheters, described in greater detail below. As such, in the manifold 18 of the aspiration catheter, two of the ports of the manifold, specifically the central port and one of the offset ports, are in fluid communication with the aspiration lumen 7. Central port 11b is characterized by the presence of Touhy-Borst valve 15b or an analogous structure. Touhy-Borst valves suitable for use in medical devices, e.g., catheters, are known in the art and described in U.S. Patent No. 5,320,613 and 5,795,307 .

Also present on the catheter shown in Fig. 1A is a strain relief 18a. The strain relief protects the proximal end of tube 16 from damage and gives strength to the transition between the tube and manifold 18. The strain relieve locally increases the stiffness of tube 16 to provide a moderated step-wise increase in stiffness from the relatively more flexible tube to the stiffer manifold member. The length of the strain relieve may vary from about 5 to about 40 mm, but is usually about 20 to about 30 mm in length. Suitable materials for strain relief 18a include fluorinated ethylene-propylene or a similar medical grade polymer.

The embodiment shown in Fig. 1A includes three-way stop cocks attached to each of the offset ports. These elements provide for further control of fluid flow into the lumens of the device and/or the introduction of two separate fluids into the same lumen of the device, e.g., an imaging solution into the aspiration lumen and aspiration of fluid through the aspiration lumen, depending on the state of the three -way stop-cock.

For convenience during use of the aspiration catheter, each of the ports of the four-port manifold may be uniquely identified, e.g., color coded, so that it is readily apparent as to the element that the port should be connected during use of the device, e.g. vacuum source, balloon inflation means, etc. For example, one of the offset ports may have a yellow band, one may have a green band, one may have a blue band and one may have a red band, thereby uniquely identifying the ports of the four-port manifold.

### Total Occlusion Multilumen Catheter

Also provided by the subject invention are multilumen delivery catheters that are designed to be inserted inside the aspiration lumen and used to flush a vascular site that is characterized by the presence of a total vascular occlusion, e.g., as described in greater detail below. These fluid "delivery" catheters are identified herein as total occlusion catheters. In general, the total occlusion catheters are characterized in that the multilumen tube separating the proximal and distal ends is a two-lumen tube. These total occlusion catheters are further characterized in that the multiport manifold is preferably a two-port manifold, where one of the ports has a luer valve or analogous element, as described above, and the other port has a Touhy-Borst valve or analogous element.

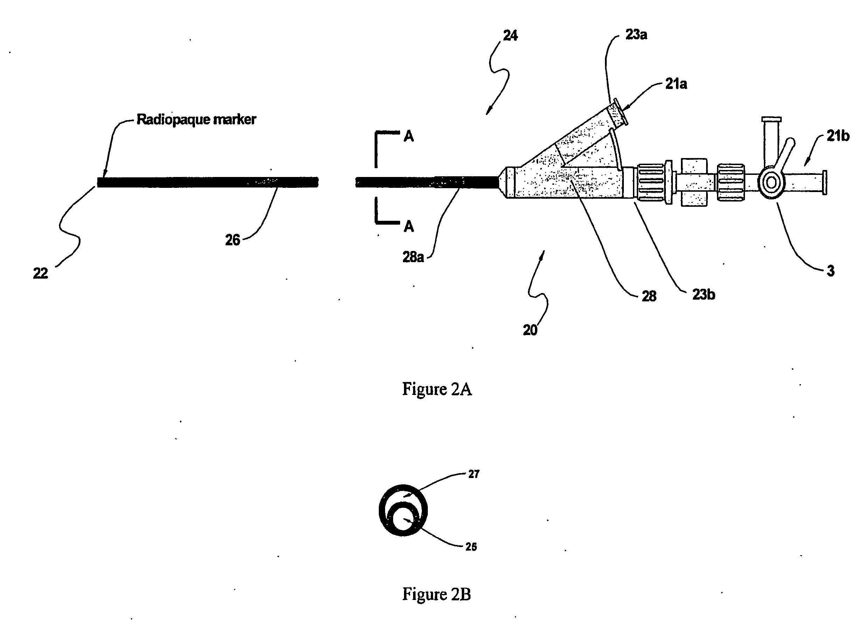

Figs. 2A and B provide a representation of a total occlusion catheter according to the subject invention. As shown in Fig. 2A, total occlusion catheter 20 includes proximal end 24 and distal end 22 separated by elongated tubular member 26. Elongated tubular member 26 is sufficiently long to provide for access of the distal end to the target vascular site upon introduction into the host vascular system via a remote entry site of the vascular system. Typically, the length of elongate member 26 ranges from about 90 to about 210 cm, usually from about 100 to about 190 cm and more usually from about 110 to about 150 cm. The outer diameter of the tubular member 26 is such that it may be slidably positioned inside the aspiration lumen of the aspiration catheter, as described above. Typically, the outer diameter of element 26 ranges from about 1.5 to about 6.0 Fr, usually from about 2.5 to about 5.0 Fr.

Tubular member 26 is a two-lumen tubular member, where the lumens of the catheter are non-coaxial. In the embodiment depicted in Figures 2A and 2B, each of the lumens, 25 and 27, open at the distal end of catheter 20. One of the lumens, e.g., 27, is designed to carry a dissolution fluid and the other lumen, 25 is designed to carry a guidewire. The configuration of the each of the lumens in the embodiment shown in Fig. 2A can be seen in Fig. 2B, which is a cross section of tubular member 26 taken at line A-A shown in Fig. 2A. As can be seen in the representative embodiment, guidewire lumen 25 has a substantially circular configuration while dissolution fluid delivery lumen 27 has a crescent configuration so as to share a common wall or border with a substantial portion of lumen 25, where by "substantial portion" is meant at least 25%, usually at least 35% and more usually at least 50% of the circumference of lumen 25, where in certain embodiments this portion may be much higher, e.g., 60%, 75% etc. Lumen 25 is typically employed for conveying the dissolution fluid while lumen 27 is typically employed for a guidewire.

The inner diameter of lumen 25 typically ranges from about 0.2 to about 1.0 mm, usually from about 0.2 to about 0.7 mm and more usually from about 0.3 to about 0.4 mm, so as to provide for a cross-sectional area of about 0.03 to about 0.8 mm², usually from about 0.03 to about 0.4 mm² and more usually from about 0.07 to about 0.2 mm². The cross-sectional area of lumen 27 typically ranges from about 0.03 to about 0.8 mm², usually from about 0.03 to about 0.4 mm² and more usually from about 0.07 to about 0.2 mm². While the configuration of Fig. 2B shows a circular and crescent shaped lumen, other configurations are also possible, e.g., two circular lumens, to semicircular lumens, etc.

Also present on the preferred total occlusion catheter at proximal end 24 is two-port manifold 28. Two-port manifold 28 includes side or offset port 21 a and central port 21b. Port 21a includes female luer valve 23a while port 21b includes Touhy-Borst valve 23b. During use, side or offset port 21 a is typically in fluid communication with a source of dissolution fluid attenuating fluid, e.g., buffer, while central port 21 b is typically sealed around a guidewire.

Also present on the catheter shown in Fig. 2A is a strain relief 28a. It functions as described in connection with strain relief 18a. In the embodiment shown in Fig. 2A, also present is three-way stop cock attached to the central port which provides for further control of fluid flow into the central lumens of the device, the introduction of two separate fluids into the same lumen of the device and/or the introduction of separate fluids for intermittent flushing, e.g. with heparinized saline, typically used to keep lumens clear and prevent clotting.

For convenience during use of the aspiration catheter, each of the ports of the two port manifold may be uniquely identified, e.g., color coded, so that it is readily apparent as to the element that the port should be connected during use of the device, e.g. dissolution fluid reservoir, dissolution fluid attenuating fluid reservoir, etc. For example, one of the offset ports may have a yellow band and one may have a red band, thereby uniquely identifying the ports of the manifold.

### Partial Occlusion Catheter

Also provided by the subject invention are multilumen catheters that are designed to be inserted inside the aspiration lumen and used to flush a vascular site that is characterized by the presence of a partial vascular occlusion. These catheters are identified herein as partial occlusion catheters. In general, the partial occlusion catheters are characterized in that the multilumen tube separating the proximal and distal ends is a three-lumen tube. These partial occlusion catheters are further characterized in that the multiport manifold is a three-port manifold, where two of the ports has a luer valve or analogous structure and the third port has a Touhy-Borst valve or analogous structure for making a sealing engagement with a guidewire inserted therethrough. In addition to the above features, the partial occlusion catheter also includes a balloon or analogous vessel occlusion means at its distal end and at least one port, and often multiple ports, proximal to the balloon, where these infusion ports are in fluid communication with one of the lumens.

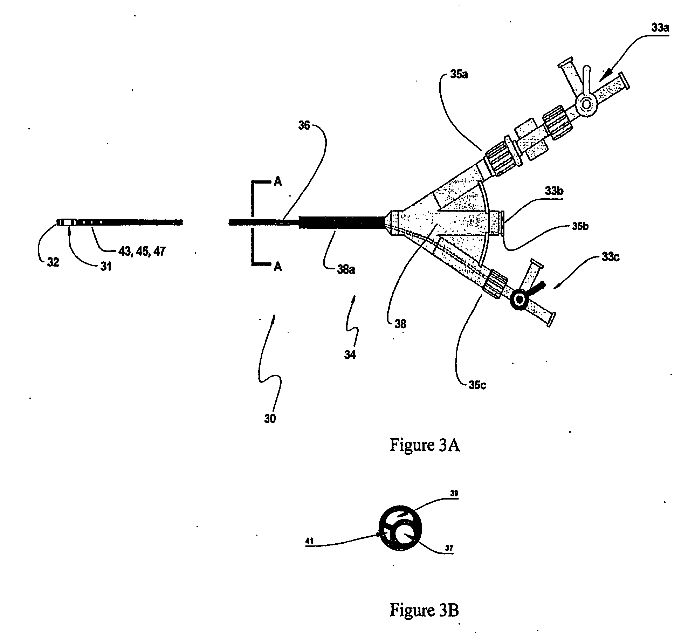

Figs. 3A and B provide a representation of a partial occlusion catheter according to the subject invention. As shown in Fig. 3A, partial occlusion catheter 30 includes distal end 32 and proximal end 34 separated by three lumen tube 36. The length of the tubular member 36 generally ranges from about 90 to about 250 cm, usually from about 100 to about 230 cm and more usually from about 110 to about 190 cm. The outer diameter of the tubular member 36 is such that the partial occlusion catheter 30 may be slidably positioned in the aspiration lumen of the aspiration catheter. As such, the outer diameter of tubular member 36 typically ranges from about 0.5 to about 2.0, usually from about 0.8 to about 1.6 mm.

Located at the distal end of catheter 30 is balloon 31 or analogous occlusion means. The balloon is generally one that is inflatable to a diameter ranging from about 2 to about 15 mm, usually about 5 to about 10 mm, and typically has a length of from about 10 to about 30 mm, usually from about 15 to about 20 mm. Proximal to the balloon 31 are a series of ports 43, 45 and 47 which provide for fluid transfer between the outside of tube 36 and the fluid delivery lumen located inside the device, i.e., a first lumen inside the tube 36 that is carrying a dissolution fluid. The diameter of the infusion ports may vary, but typically ranges from about 0.2 to about 1.2, usually from about 0.4 to about 1.0 and more usually from about 0.5 to 0.8 mm.

A representative configuration of the three lumens of multilumen tube 36 is shown in Fig. 3B, which shows the cross section of tube 36 taken along line A-A as shown in Fig. 3A. As shown in Fig. 3B, lumen 37 has a substantially circular cross section and is designed to provide a passage for a guidewire, while lumens 39 and 41 together make up a crescent shape that shares a border with lumen 37 that extends for a substantial portion of the perimeter of lumen 37. The crescent shape made through combination of lumens 39 and 41 is divided to provide for distinct lumens 39 and 41. The diameter of lumen 37 typically ranges from about .02 to about 1.0 mm, usually from about 0.2 to about 0.7 mm and more usually from about 0.3 to about 0.4 mm to provide a cross-sectional area ranging from about 0.03 to about 0.8 mm², usually from about 0.03 to about 0.4 mm². The cross sectional area of lumen 39 typically ranges from about 0.03 to about 0.8 mm², usually from about 0.03 to about 0.4 mm² while the cross-sectional area of lumen 41 typically ranges from about 0.05 to about 0.3, usually from about 0.1 to about 0.2. In many embodiments, lumen 37 conveys a guidewire, lumen 39 conveys dissolution fluid, e.g., acid, and lumen 41 conveys inflation medium, e.g., gas or fluid, to the balloon. While one type of configuration of the various lumens is shown, other non-coaxial configurations are also possible, e.g., three separate circles, a circle and two distinct crescent shapes, etc., where all of these potential configurations are within the scope of the invention.

Located at the proximal end 34 of catheter 30 is three-port manifold 38. Three- port manifold 38 includes two offset or side ports 33a and 33c, which flank central port 33b. The two side ports each include a female luer valve, 35a, 35b and 35c. The central port 33b includes Touhy-Borst valve 35b for producing a sealing engagement with a guidewire inserted therethrough. Offset port 33a is typically in fluid communication with a source of dissolution fluid. Offset port 33c is typically in fluid communication with a balloon inflation means, e.g., a syringe filled with a gas or liquid.

Also present on the catheter shown in Fig. 3A is a strain relief 38a. It functions in the manner of strain relief 18a and 28a.

In the embodiment shown in Fig. 3A, also present are three-way stop cocks attached to each of the offset ports, which elements provide for further control of fluid flow into the lumens of the device, the introduction of two separate fluids into the same lumen of the device and/or the introduction of separate fluids for intermittent flushing, e.g. with heparinized saline, typically used to keep lumens clear and prevent clotting, depending on the state of the three way stop-cock.

For convenience during use of the aspiration catheter, each of the ports of the three port manifold may be uniquely identified, e.g., color coded, so that it is readily apparent as to the element that the port should be connected during use of the device, e.g. vacuum dissolution fluid, dissolution fluid attenuating fluid, balloon inflation means, etc.

### SYSTEMS

Also provided by the subject invention are systems for flushing a vascular site with two different fluids. By flushing a vascular site is meant introducing fluid into and removing fluid from a vascular site at substantially the same time such that the vascular site remains substantially stable in terms of pressure, e.g., is isobaric, where the pressure changes that occur in the vascular site do not exceed in magnitude a value of about 50 mm Hg and usually do not exceed about 30 mm Hg. Accordingly, maximum pressure will typically remain below about 400 mm Hg, preferably about 100 mm Hg.

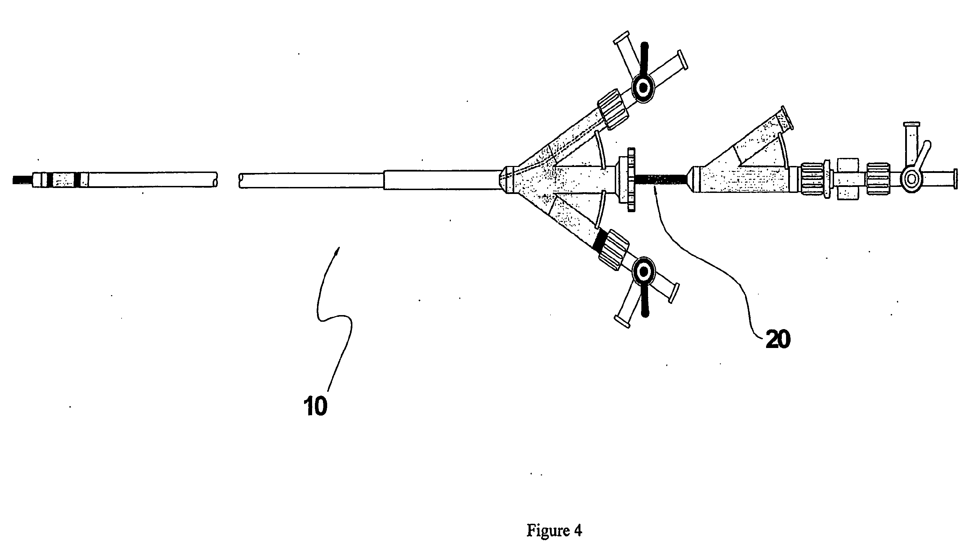

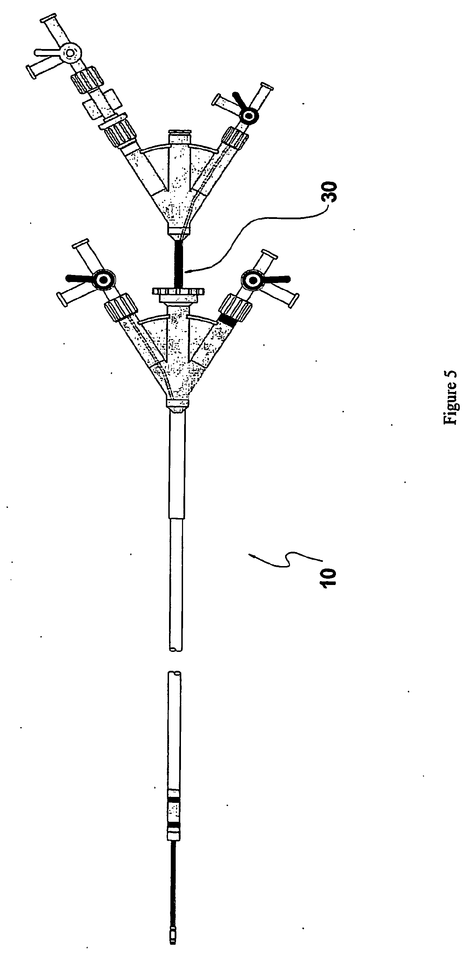

The subject systems are characterized by having an aspiration catheter and one of the total or partial occlusion catheters of the subject invention in a nested configuration, such that the total or partial occlusion catheter is slidably positioned inside the aspiration lumen of the aspiration catheter. Fig. 4 provides a representation of a system designed for use with a total occlusion, where the system is made up of a total occlusion catheter 20 inserted into the aspiration lumen of an aspiration catheter. Fig. 5 provides a representation of a system designed for use in the treatment of a partial occlusion, where the system is made up of a partial occlusion catheter inserted into the aspiration lumen of an aspiration catheter. Because the central manifold port of the aspiration catheter may have a Touhy-Borst valve, a fluid seal can be produced at the interface between the aspiration manifold central port and the outer surface of the partial or total occlusion catheter when inserted into the aspiration lumen of the aspiration catheter.

In using the total occlusion catheter system of the subject invention, one of the offset ports of the aspiration manifold is often in fluid communication with a syringe balloon inflation means, another of the offset ports of the aspiration manifold is in fluid communication with a vacuum source to provide the force for removing fluid from the vascular site via the aspiration lumen, and the third offset port is in fluid communication with the a source of dissolution fluid attenuating fluid e.g., an ENDoFLATOR®, etc. The offset port of the total occlusion manifold is in fluid communication with a source of dissolution fluid, e.g., an ENDoFLATOR®, etc., while a guidewire is often present in the central lumen and inserted through the central port.

In using the partial occlusion catheter system according to the subject invention, one of the offset ports of the aspiration manifold is often in fluid communication with a balloon inflation mechanism, e.g., a fluid or gas filled syringe, a second of the offset ports of the aspiration manifold is in fluid communication with a vacuum source, and the third offset port is in fluid communication with a source of dissolution fluid attenuating fluid, e.g., an ENDoFLATOR® filled with dissolution fluid attenuating fluid. The central port of the partial occlusion manifold is sealed around a guidewire, one of the offset ports is in fluid communication with a source of dissolution fluid, e.g. an ENDoFLATOR® filled with dissolution fluid, and the other offset port is in fluid communication with a balloon inflator, e.g. a fluid or gas filled syringe. In use, each of the catheters are filled with the respective solutions for which sources are provided. Furthermore, balloons provided will be at least partially filled, together with adjacent feed lumen in use. The aspiration lumen may also be subject to at least partial vacuum.

As summarized above, during use a guidewire is present in the delivery catheter that is, in turn, present in the aspiration lumen of the aspiration catheter. Any convenient guidewire may be present. Representative guidewires include, but are not limited to, those guidewires described in U.S. Patent Nos. 6,007,514; 5,980,471; 5,957,865; 5,938,609; 5,931,819; 5,916,178; 5,908,395; 5,902,254; 5,865,767; 5,827,201; 5,788,654; 5,772,609; 5,769,796; 5,755,695; 5,749,837; 5,682,897; 5,660,180; 5,636,642; 5,606,981; 5,599,492; 5,596,996; 5,558,093; 5,546,948; 5,520,189; 5,507,301; 5,497,782; D363,776; 5,460,187; 5,441,497; 5,437,288; 5,427,118; 5,421,349; 5,411,033; 5,409,015; 5,368,035; 5,341,818; 5,339,833; 5,313,967; 5,303,714; RE34,466; 5,265,622; 5,238,005; 5,184,621; 5,167,239; 5,147, 317; 5,144, 959; 5,111,829; 5,107, 852; 5, 095, 915; 5,095,911 5,084,022; 5,069,226; 5,063,935; 4,966,163; 4,953,553; 4,875,489; 4,827,941; 4,811,743; 4,676,249; 4,534,363; 4,080,706 and 4,003,369 . Of interest in certain applications is the use of hollow guidewires, where such guidewires include, but are not limited to, those guidewires described in U.S. Patent Nos.: 5,569,197; 6,068,623; 6,217,567; and 6,059,767 .

### METHODS

The subject systems find use in applications where it is desired to simultaneously flush a vascular target site with two different fluids. As mentioned above, by flush it is meant that the fluid is introduced into the vascular site and removed from the vascular site in a manner such that the vascular site remains at substantially constant pressure. However, the pressure throughout the treatment site is predicted to be, on average, lower than the backbleed pressure, i.e., the blood pressure on the proximal side of the aspiration catheter balloon. While the subject systems can, in principle, be employed to flush a vascular site with any two fluids, they are particularly suited for use in applications where chemical tissue ablation at a target vascular site is desired. As such, the subject systems find particular use in the treatment of vascular lesions or obstructions, where the target lesions or obstructions may be organic, inorganic or composite structures of both organic and inorganic components. In such embodiments, the systems are used to flush the target vascular site, and therefore the lesion or obstruction located therein, with a dissolution fluid and dissolution fluid attenuating fluid.

In these examples of the subject methods, the first step is generally to provide for an entry site for the catheter into the vascular system of the host. Entry is typically provided by placement of an introducer sheath at a convenient location, e.g. leg etc., as is known in the art. A guidewire is then inserted through the entry sheath and its distal end is placed at the target vascular site. The aspiration catheter with a dilator present in the central lumen is then moved over the guidewire, where the guidewire generally passes through the central lumen, so that the distal end of the aspiration catheter reaches the target vascular site. Following placement of the distal end of the aspiration catheter at the target vascular site and subsequent deployment of the vascular occlusion means, the dilator is removed and replaced with either the partial or total occlusion catheter inserted through the Touhy-Borst valve of the aspiration manifold central port and into the aspiration lumen. This catheter insert is then moved through the lumen, over the guidewire that is still present, until the distal end of the insert is beyond the distal end of the aspiration catheter. In many embodiments, the distal end of the insert will extend some distance beyond the distal end of the aspiration catheter, where this distance typically does not exceed about 20 cm and usually does not exceed about 5 mm.

Upon positioning of the catheter system as described above and deployment of any vascular occlusion means, the dissolution fluid and dissolution fluid attenuating fluid are introduced into the vascular site via the appropriate lumens inside the delivery and aspiration catheters and fluid is removed from the vascular site via the aspiration lumen of the aspiration catheter, and specifically through the annular space present in the system bordered by the inner wall of the aspiration lumen and the outer wall of the catheter insert. The nature of the dissolution fluid and the dissolution fluid attenuating fluid necessarily depends on the nature of the target lesion to be treated. For example, for organic matter comprising lesions, organic matter dissolution fluids (and their companion attenuating fluids) are of interest, such as those described in U.S. Application Patent No. 09/528,576; the disclosure of which is herein incorporated by reference. In other embodiments where the target lesion comprises inorganic matter, acidic dissolution solutions and their companion buffer attenuating fluids are of interest, such as those described in WO 00/03651; the disclosure of the priority document of which is herein incorporated by reference. See e.g., WO 00/03651; WO 01/13985; WO 01/15767; WO 01/39783; WO 01/70320; and WO 02/15958 .

In many examples , the dissolution fluid employed in the subject methods is an inorganic matter dissolution solution. In many of these examples, the inorganic matter dissolution fluid is an acidic dissolution fluid. A variety of different types of acidic dissolution solutions may be employed in the subject methods. The acidic treatment solutions that find use in the subject methods generally have a pH of less than about 6.5, where the pH is usually less than about 4.0 and more usually less than about 3.0. In many preferred embodiments, the pH ranges from 0 to 2, and usually 0 to 1. The acidic treatment solution can include a number of different types of acids, where the acids may or may not include a hydrocarbon moiety, i.e., a hydrogen bonded directly to a carbon atom. Suitable acids that lack a hydrocarbon moiety include halogen acids, oxy acids and mixtures thereof, where specific acids of interest of this type include, but are not limited to, hydrochloric, nitric, sulfuric, phosphoric, hydroboric, hydrobromic, carbonic and hydroiotic acids. For such acids, the acid can be a concentrated acid, or can be diluted. Upon dilution, the concentration of an inorganic acid will generally be from about 10 N to about 0.01 N, preferably between 5 N to 0.1 N. Also of interest are acids that include a hydrocarbon moiety, where such acids include, but are not limited to, any organic acid of one to six (C₁ to C₆) carbons in length. Organic acids of this type include, but are not limited to, formic, acetic, propionic, maleic, butanoic, valeric, hexanoic, phenolic, cyclopentanecarboxylic, benzoic, and the like. For an organic acid, the acid can be in concentrated form, or can be diluted. The acidic treatment solution can be composed of either a monobasic or a polybasic acid. Acids are "monobasic" when they have only one replaceable hydrogen atom and yield only one series of salts (e.g., HCl). Acids are "polybasic" when they contain two or more hydrogen atoms which may be neutralized by alkalies and replaced by organic radicals.

In many examples, the acid solution is hypertonic, by which is meant that the osmolarity of the solution is greater than that of whole blood, i.e. the osomolarity is greater than 300 mosmol. The solution may be rendered hypertonic by including any convenient component or components in the solution that provide for the desired elevated osmolarity.

Any convenient agent that is capable of increasing the osmolarity of the solution may be employed, where suitable agents include salts, sugars, and the like. In many embodiments, the agent that is employed to render the solution hypertonic is one or more, usually no more than three, and more usually no more than two, different salts. Generally, the salt concentration in these embodiments of the solution is at least about 100 mosmol, usually at least about 200 mosmol and more usually at least about 300 mosmol, where the concentration may be as high as 3000 mosmol or higher, depending on the particular salt being employed to render the solution hypertonic, where the solution may be saturated with respect to the salt in certain embodiments. Salts that may be present in the subject solutions include: NaCl, MgCl₂, Ringers, etc. where NaCl is preferred in many examples.

Of particular interest in many examples is the use of a hydrogen chloride solution. In hydrogen chloride solutions that find use in the subject method, the concentration of HCl in the solution ranges from about 0.001 to 1.0 N, usually from about 0.01 to 1.0 N and more usually from about 0.1 to 1.0 N. In many examples, the hydrogen chloride solution will further include one or more salts which ma the solution hypertonic, as described above. In certain preferred examples, the salt is NaCl, where the concentration of NaCl in the solution is at least 0.05 M, usually at least 0.10 M, and more usually at least 0.15 M, where the concentration may be as high as 0.25 M or higher. In certain examples, the solution will be saturated with NaCl.

Of particular interest are aqueous hydrogen chloride solutions that consist of water, hydrogen chloride and NaCl. The concentration of hydrogen chloride in these solutions of particular interest ranges from about 0.01 to 1.0 N, usually from about 0.05 to 0.5 N and more usually from about 0.075 to .25 N. The concentration of NaCl in these solutions of particular interest ranges from about 0.05 to 0.25 M, usually from about 0.05 to 10 M.

In certain examples, one or more of the delivery fluids is present at a temperature that is less than room temperature. For example, in certain examples, the one or more treatment solutions, as described above, is present at a temperature ranging from about 0 to about 20 °C, sometimes from about 0 to 15°C, e.g., from about 0 to 10°C. Such examples include applications where it is desired to limit restinosis by employing reduced temperature, e.g., cold, solutions.

The target vascular site is flushed with the dissolution and dissolution fluid attenuating fluids for a period of time sufficient to result in the desired amount of treatment, e.g., target lesion size reduction, enhancement or establishment of fluid flow through the target site, etc. Following the desired amount of treatment, the system is removed from the host. More specific detail regarding the methods in which the subject systems find use can be found in U.S. Patent No. 09/528, 576; and publication no. WO 00/03651 .

In certain examples, external energy is applied to the target aortic valve to promote mechanical break-up of the calcified deposits into particles or debris that can be easily removed from the vascular site. Any means of applying external energy to the aortic valve may be employed. As such, jets or other such means the device which are capable of providing varying external forces to the target deposits cause the target deposit to break up or disrupt may be employed. Of particular interest in many embodiments is the use of sonic energy, e.g., infrasound, ultrasound, etc. The sonic energy, e.g., ultrasound, can be applied during the entire time of contact of the cardiovascular tissue with the acidic treatment solution, or it can be applied for only part of the treatment period. In one embodiment, the sonic energy is applied for several short periods of time while the dissolution treatment solution is contacted with the target occlusion. Sonic energy elements that are suitable for use with the subject methods are known and readily available to those of skill in the art. For example, U.S. Patent Nos.: 5,695,460; 5,997,497; 6,047,700; 6,083,573; 6,113,570; and 6,308,714; as well as the United States Patents cited therein; all describe various ultrasound technologies for delivering ultrasound to a physiological, including cardiovascular, site, where such technologies include transdermal delivery technologies. . Another means that may be employed to apply external energy to the lesion during the dissolution process is to use a mechanical means of applying external energy. Mechanical means of interest include moving structures, e.g. rotating wires, guidewires, which physically contact the target lesion and thereby apply physical external energy to the target lesion.

In certain examples, as described above, the local environment of the target lesion is rendered substantially bloodless prior to introduction of the acidic dissolution fluid. In these examples, the isolation system is deployed to physically isolate the local environment from the remainder of the circulatory system and then the local environment is flushed with a physiologically acceptable solution, such that substantially all of the blood present in the solution is removed. Typically, a washing solution will be employed in this step of rendering the local environment bloodless. Examples of washing solutions that may find use in these examples include: water for injection, saline solutions, e.g. Ringer's, phosphate buffered saline, or other physiologically acceptable solutions. The washing solution may include an anti-clotting factor in many examples, where anticlotting factors of interest include heparin and the like. The washing solution can also contain chelating agents.

In addition, it may be convenient to monitor or visualize the vascular site prior to or during treatment. A variety of suitable monitoring means are known to those of skill in the art. Any convenient means of invasive or noninvasive detection and/or quantification may be employed. Such means include plain film roentgenography, coronary arteriography, fluoroscopy, including digital subtraction fluoroscopy, cinefluorography, conventional, helical and electron beam computed tomography, intravascular ultrasound (IVUS), magnetic resonance imaging, transthoracic and transesophageal echocardiography, rapid CT scanning, antioscopy and the like. Any of these means can be used to monitor the vascular site before, during or after contact with the dissolution fluid.

In many examples, an imaging agent is employed, where the imaging agent may or may not be present in the acidic dissolution solution. Imaging agents of particular interest include: non-ionic imaging agents, e.g. CONRAY™, OXILAN™, and the like

In certain examples as mentioned above, the guidewire over which the delivery and aspiration catheters are present is a hollow guidewire. In these examples, the guidewire may be employed for a number of additional functions. For example, where a hollow guidewire is employed in the total occlusion delivery catheter system, the guidewire may be employed to convey additional dissolution fluid to the target site. In other embodiments where the hollow guidewire is employed in the partial occlusion catheter, it may be employed to convey a perfusate, such as an oxygen bearing medium, to a region beyond the distal balloon and isolated environment bordered thereby. Perfusates of interest include, but are not limited to: (1) the oxygen gas supersaturated fluids as described in U.S. Patent Nos. 6,315,754; 5,693,017; 5,797,874; and the like.

While the methods are suitable for use in the treatment of any type of vascular lesion, the methods are particularly suited for treatment of coronary vascular lesions, specifically coronary vascular calcified lesions, such as lesions that might be found in coronary arteries, which lesions may be total or partial occlusions, as described above. By using the subject devices suitably dimensioned to be positioned inside of the coronary vascular structure to be treated, coronary vascular lesions can readily be treated according to the subject invention. The subject methods may be employed in conjunction with other coronary vascular treatments, e.g., stent deployment, etc.

### KITS

Also provided by the subject invention are kits for use in flushing a vascular site with two fluids. The subject kits at least include the components of a partial occlusion or total occlusion catheter system according to the subject invention. As such, the kits include an aspiration catheter and at least one of a partial occlusion catheter and a total occlusion catheter. In many embodiments, the kits include both a partial occlusion catheter and a total occlusion catheter. The subject kits may also include a dissolution fluid and/or dissolution fluid attenuating fluid, or components/precursors thereof, where representative dissolution fluids and dissolution fluid attenuating fluids are disclosed above. The dissolution fluids and dissolution fluid attenuating fluids or component(s) thereof are present in the kit in a suitable container, e.g., a bottle, pouch, fluid filled an ENDoFLATOR®, etc. which is capable of serving as a storage vessel for the this component of the kit and, preferably, capable of preserving the sterility of this component of the kit, as this component of the kit is preferably sterile, e.g., medical grade.

The kits of the subject invention may also include a number of different components, which components may find use in methods in which the subject kit components are employed. One component that may be present in the subject kits is a guidewire. Any convenient type of guidewire may be present, where a number of different guidewires are known to those of skill in the art. Guidewires of interest include those described in U.S. Patent Nos. 6,007,514; 5,980,471; 5,957,865; 5,938,609; 5,931,819; 5,916,178; 5,908,395; 5,902,254; 5,865,767; 5,827,201; 5,788,654; 5,772,609; 5,769,796; 5,755,695; 5,749,837; 5,682,897; 5,660,180; 5,636,642; 5,606,981; 5,599,492; 5,596,996; 5,558,093; 5,546,948; 5,520,189; 5,507,301; 5,497,782; D363,776; 5,460,187; 5,441,497; 5,437,288; 5,427,118; 5,421,349; 5,411,033; 5,409,015; 5,368,035; 5,341,818; 5,339,833; 5,313,967; 5,303,714; RE34,466; 5,265,622; 5,238,005; 5,184,621; 5,167,239; 5,147,317; 5,144,959; 5,111,829; 5,107,852; 5,095,915; 5,095,911 5,084,022; 5,069,226; 5,063,935; 4,966,163; 4,953,553; 4,875,489; 4,827,941; 4,811,743; 4,676,249; 4,534,363; 4,080,706 and 4,003,369 . Also of interest are dilators for use in creating entries into the vascular system of the host.

Additional optional components that may be present in kits of the subject invention include various fluids and solutions in addition to the dissolution fluid and dissolution fluid attenuating fluid described above. Additional fluids that may be present include: organic matter dissolution fluids, wash or rinsing fluids, imaging agent fluid mediums that include an imaging agent, such as a non-ionic imaging agents, e.g., CONRAY™, OXILAN™, fluids containing one or more pharmacological agents, e.g., agents that promote healing, reduce inflammation, and the like; etc.

Other components that may be present in the subject kits include one or more additional components and accessories for use with the fluid delivery means present in the kit, including tubing for connecting the various catheter components with fluid reservoirs, syringes, pumps, etc., connectors, stop-cocks, dilators, insertion sheaths, vacuum regulators, negative pressure or vacuum generators/sources, luer valve adapters, etc.

In addition to above mentioned components, the subject kits typically further include instructions for using the components of the kit to flush a vascular site with two different fluids, e.g., to flush a vascular site with a dissolution fluid and a dissolution fluid attenuating fluid. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

It is evident from the above discussion that the catheters and systems that are made up of the same provide a reliable and convenient way to flush a target vascular site with two different fluids. Because the catheter devices have a design that allows them to be produced via extrusion technology using polymeric materials, they may be economically produced and the number of different working elements is kept to a minimum. In addition, the catheter design allows the catheters to be scaled to treat a wide variety of different type of vascular lesions, including coronary vascular lesions. In view of these factors and such others discussed above, the subject invention represents a significant contribution to the art.

The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

## Claims

1. A system for flushing a vascular site with a fluid, said system comprising:
(I) a three-lumen aspiration catheter (10) comprising:
(A) a proximal end (14);
(B) a distal end (12); and
(C) a four-port manifold (18) located at said proximal end, wherein:
(i) said four-port manifold comprises three side or offset ports (11a, 11b, 11d) and a central port (11b), wherein the side or offset ports (11a, 11b, 11d) have a luer-type connector (15a, 15c, 15d) and the central port (11b) comprises a sealing element for producing a sealing engagement around a tubular element inserted therethrough, wherein one of said ports of said four-port manifold of said multilumen aspiration catheter is in fluid communication with a dissolution solution attenuating solution fluid reservoir , and
(ii)said proximal and distal ends are separated by a non-coaxial three-lumen tube (16);
wherein the first lumen is the aspiration lumen (7), which opens at the distal end to provide a fluid entry site for fluid from the target vascular site to flow into the lumen (7)
wherein the second lumen is an inflation lumen (9) which runs the length of the aspiration catheter from the proximal end to vessel occlusion means (13),
wherein the third lumen (5) is employed for delivering a dissolution fluid attenuating fluid to the target vascular site
wherein one of the offset ports (11a) of the manifold (18) is in fluid communication with the second lumen (9),
wherein the central port (11b) is in fluid communication with the aspiration lumen
wherein one of the offset ports (11c) of the manifold (18) is in fluid communication with the aspiration lumen (7),
wherein one of the offset ports (11d) of the manifold (18) is in fluid communication with the third lumen (5),
and,
(II) a multilumen delivery catheter inserted inside the aspiration lumen (7) of said first three lumen catheter via said central port (11b), wherein said multilumen delivery catheter is a catheter selected from the group consisting of:
(A) a first two-lumen delivery catheter, being a total occlusion catheter (20), comprising:
(i) a proximal end (24);
(ii) a distal end (22); and
(iii) a two-port manifold (28) located at said proximal end (24), which manifold (28) includes a side or offset port (21a) comprising a luer type connector and a central port (21b) suitable for sealing around a guidewire, and wherein said proximal and distal ends are separated by a non-coaxial two-lumen tube (26), wherein one of the lumens (27) is designed to carry a dissolution fluid and the other lumen (25) is designed to carry a guidewire, and
(B) a second three-lumen delivery catheter being a partial occlusion catheter (30) comprising:
(i) a proximal end (34);
(ii) a distal end (32);
(iii) occlusion means (31); and
(iii) a three-port manifold (38) located at said proximal end, wherein, at least two ports of said three-port manifold comprise a luer-type connector, which manifold (38) includes two offsets or side ports (33a, 33c) and a central port (33b) sealed around a guidewire, and wherein said proximal and distal ends are separated by a non-coaxial three-lumen tube (36), wherein one of the lumens (37) is suitable for conveying the guidewire, one of the lumens (39) is suitable for conveying dissolution fluid and one of the lumens (41) is suitable for conveying inflation medium for the occlusion means.

2. The system according to Claim 1, wherein said delivery multilumen catheter is said first two-lumen delivery catheter.

3. The system according to Claim 1, wherein said delivery multilumen catheter is said second three-lumen delivery catheter.

4. The system according to any of Claims 1 to 3, wherein said sealing element of said fourth port of said aspiration catheter is suitable for producing a sealing engagement with either said first or second delivery multilumen catheter when inserted therethrough.

5. The system according to any of Claims 1 to 4, wherein the offset port (11c) of the manifold (18) is in fluid communication with a negative pressure source.

6. The system according to any of Claims 1 to 5, wherein one of said ports of said manifold of said multilumen delivery catheter is in fluid communication with a dissolution fluid reservoir.

7. A kit for use in flushing a vascular site with fluid, said kit comprising a system according to any of the preceding claims and instructions for the use thereof.

## Patentansprüche

1. System zum Spülen einer Gefäßstelle mit einem Fluid, wobei das System aufweist:
(I)einen Drei-Lumen-Saugkatheter (10) mit:
(A) einem proximalen Ende (14),
(B) einem distalen Ende (12) und
(C) einem Verteiler (18) mit vier Anschlüssen, der an dem proximalen
Ende angeordnet ist, wobei:
(i) der Verteiler mit vier Anschlüssen drei Seiten- oder Versatzanschlüsse (11a, 11b, 11d) und einen Zentralanschluss (11b) aufweist, wobei die Seiten- oder Versatzanschlüsse (11a, 11b, 11d) einen Verbinder (15a, 15c, 15d) vom Luer-Typ aufweist und der Zentralanschluss (11b) ein Dichtelement zum Erzeugen eines Dichtungseingriffs um ein darin eingefügtes rohrförmiges Element herum aufweist, wobei einer der Anschlüsse des Vierfachverteilers des Multi-lumensaugkatheters ausgebildet ist, um in Fluidverbindung mit einem Lösungsfluid-Dämpfungsfluidresservoir zu stehen, und
(ii) das proximale Ende und das distale Ende durch ein nicht koaxiales Dreilumenrohr (16) getrennt sind,
wobei das erste Lumen das Sauglumen (7) ist, das sich am distalen Ende öffnet, um eine Fluideingangsstelle für Fluid von der Zielgefäßstelle zu bilden, um in das Lumen (7) zu fließen,
wobei das zweite Lumen ein Inflationslumen (9) ist, das über die Länge des Saugkatheters von dem proximalen Ende zu Gefäßverschlussmitteln (13) verläuft,
wobei das dritte Lumen (5) zum Liefern eines Lösungsfluids-Dämpfungsfluids an die Zielgefäßstelle aus geeignet ist,
wobei einer der Versatzanschlüsse (11a) des Verteilers (18) in Fluidverbindung mit dem zweiten Lumen (9) ist,
wobei der Zentralanschluss (11b) in Fluidverbindung mit dem Sauglumen ist,
wobei einer der Versatzanschlüsse (11c) des Verteilers (18) in Fluidverbindung mit dem Sauglumen (7) ist,
wobei einer der Versatzanschlüsse (11d) des Verteilers (18) in Fluidverbindung mit dem dritten Lumen (5) ist, und
(II) einen Multilumen-Zufuhrkatheter, der innerhalb des Sauglumens (7) des ersten Dreilumenkatheters über den Zentralanschluss (11b) eingefügt ist, wobei der Multilumen-Zufuhrkatheter ein Katheter ist, der aus der Gruppe ausgewählt ist, die besteht aus:
(A) einem ersten Zweilumenzufuhrkatheter, der ein Gesamtverschlusskatheter (20) ist, mit:
(i) einem proximalen Ende (24),
(ii) einem distalen Ende (22) und
(iii) einem Zweianschlussverteiler (28), der an dem proximalen Ende (24) angeordnet ist, wobei der Verteiler (28) einen Seiten- oder Versatzanschluss (21a) aufweist, der einen Verbinder vom Luer-Typ aufweist, und einen Zentralanschluss (21b), der für das Abdichten um einen Führungsdraht herum ausgebildet ist, und wobei das proximale und das distale Ende durch ein nicht koaxiales Zweilumenrohr (26) getrennt sind, wobei eines der Lumen (27) zur Aufnahme eines Lösungsfulids ausgebildet ist und das andere Lumen (25) zur Aufnahme eines Führungsdrathes ausgebildet ist, und
(B) einem zweiten Dreilumenzufuhrkatheter, der ein Teilverschlusskatheter (30) ist, mit:
(i) einem proximalen Ende (34),
(ii) einem distalen Ende (32),
(iii) Verschlussmitteln und
(iii) einem Dreianschlussverteiler (38), der an dem proximalen Ende angeordnet ist, wobei zumindest zwei Anschlüsse des Dreianschlussverteilers einen Verbinder vom Luer-Typ aufweist, wobei der Verteiler (38) zwei Versatz- oder Seitenanschlüsse (33a, 33c) und einen Zentralanschluss (33b) aufweist, der um einen Führungsdraht herum abgedichtet ist, und wobei das proximale und das distale Ende durch ein nicht koaxiales Dreilumenrohr (36) getrennt sind, wobei eines der Lumen (37) zum Fördern des Führungsdrahtes ausgebildet ist, eines der Lumen (39) zum Führen von Lösungsfluid geeignet ist und eines der Lumen (41) zum Führen eines Inflationsmittels der Verschlussmittel geeignet ist.

2. System nach Anspruch 1, wobei der Zufuhrmultilumenkatheter der erste Zweilumenzufuhrkatheter ist.

3. System nach Anspruch 1, wobei der Zufuhrmultilumenkatheter der zweite Dreilumenzufuhrkatheter ist.

4. System nach einem der Ansprüche 1 bis 3, wobei das Dichtelement des vierten Anschlusses des Saugkatheters geeignet ist zur Erzeugung eines Dichteingriffs mit entweder dem ersten oder dem zweiten Zufuhrmehrlumenkatheter, wenn er dadurch eingebracht wird.

5. System nach einem der Ansprüche 1 bis 4, wobei der Versatzanschluss (11c) des Verteilers (18) in Fluidverbindung mit einer Negativdruckquelle ist.

6. System nach einem der Ansprüche 1 bis 5, wobei einer der Anschlüsse des Verteilers des Mehrlumenzufuhrkatheters in Fluidverbindung mit einem Lösungsfluidreservoir ist.

7. Satz zur Verwendung zum Spülen einer Gefäßstelle mit Fluid, wobei der Satz ein System nach einem der vorstehenden Ansprüche und Anleitungen für seine Bedienung aufweist.

## Revendications

1. Système pour rincer un site vasculaire avec un fluide, ledit système comprenant:
(I) un cathéter d'aspiration à trois lumières (10) comprenant:
(A) une extrémité proximale (14);
(B) une extrémité distale (12); et
(C) un collecteur à quatre orifices (18) positionné au niveau de ladite extrémité proximale, dans lequel:
(i) ledit collecteur à quatre orifices comprend trois orifices latéraux ou décalés (11a, 11b, 11d) et un orifice central (11b), dans lequel les orifices latéraux ou décalés (11a, 11b, 11d) ont un connecteur de type Luer (15a, 15c, 15d) et l'orifice central (11b) comprend un élément d'étanchéité pour produire une mise en prise étanche autour d'un élément tubulaire inséré à travers ce dernier, dans lequel l'un desdits orifices dudit collecteur à quatre orifices dudit cathéter d'aspiration à plusieurs lumières est approprié pour être en communication de fluide avec un réservoir de fluide isolant de fluide de dissolution, et
(ii) lesdites extrémités proximale et distale sont séparées par un tube à trois lumières non coaxiales (16);
dans lequel la première lumière est la lumière d'aspiration (7) qui s'ouvre au niveau de l'extrémité distale pour fournir un site d'entrée de fluide pour que le fluide provenant du site vasculaire cible s'écoule dans la lumière (7),
dans lequel la deuxième lumière est une lumière de gonflage (9) qui s'étend sur la longueur de cathéter d'aspiration à partir de l'extrémité proximale jusqu'aux moyens d'occlusion de vaisseau (13),
dans lequel la troisième lumière (5) est appropriée pour délivrer un fluide isolant de fluide de dissolution sur le site vasculaire cible,
dans lequel l'un des orifices décalés (11a) du collecteur (18) est en communication de fluide avec la deuxième lumière (9),
dans lequel l'orifice central (11b) est en communication de fluide avec la lumière d'aspiration,
dans lequel l'un des orifices décalés (11c) du collecteur (18) est en communication de fluide avec la lumière d'aspiration (7),
dans lequel l'un des orifices décalés (11d) du collecteur (18) est en communication de fluide avec la troisième lumière (5),
et,
(II) un cathéter de pose à plusieurs lumières inséré à l'intérieur de la lumière d'aspiration (7) dudit premier cathéter à trois lumières via ledit orifice central (11b), dans lequel ledit cathéter de pose à plusieurs lumières est un cathéter choisi dans le groupe comprenant :
(A) un premier cathéter de pose à deux lumières, qui est un cathéter d'occlusion totale (20), comprenant :
(i) une extrémité proximale (24) ;
(ii) une extrémité distale (22) ; et
(iii) un collecteur à deux orifices (28) positionné au niveau de ladite extrémité proximale (24), lequel collecteur (28) comprend un orifice latéral ou décalé (21a) comprenant un connecteur de type Luer et un orifice central (21b) approprié pour réaliser l'étanchéité autour d'un fil guide, et dans lequel lesdites extrémités proximale et distale sont séparées par un tube à deux lumières non coaxial (26), dans lequel l'une des lumières (27) est appropriée pour transporter un fluide de dissolution et l'autre lumière (25) est appropriée pour transporter un fil guide ; et
(B) un deuxième cathéter de pose à trois lumières qui est un cathéter d'occlusion partielle (30) comprenant :
(i) une extrémité proximale (34) ;
(ii) une extrémité distale (32) ;
(iii) des moyens d'occlusion (31) ; et
(iv) un collecteur à trois orifices (38) positionné au niveau de ladite extrémité proximale, dans lequel, au moins deux orifices dudit collecteur à trois orifices comprennent un connecteur de type Luer, lequel collecteur (38) comprend deux orifices décalés ou latéraux (33a, 33c) et un orifice central (33b) étanche autour d'un fil guide, et dans lequel lesdites extrémités proximale et distale sont séparées par un tube à trois lumières non coaxiales (36), dans lequel l'une des lumières (37) est appropriée pour transporter le fil guide, l'une des lumières (39) est appropriée pour transporter le fluide de dissolution et l'une des lumières (41) est appropriée pour transporter le moyen de gonflage pour les moyens d'occlusion.

2. Système selon la revendication 1, dans lequel ledit cathéter de pose à plusieurs lumières est ledit premier cathéter de pose à deux lumières.

3. Système selon la revendication 1, dans lequel ledit cathéter de pose à plusieurs lumières est ledit deuxième cathéter de pose à trois lumières.

4. Système selon l'une quelconque des revendications 1 à 3, dans lequel ledit élément d'étanchéité dudit quatrième orifice dudit cathéter d'aspiration est approprié pour produire une mise en prise étanche avec ledit premier ou deuxième cathéter de pose à plusieurs lumières, lorsqu'il est inséré à travers ce dernier.

5. Système selon l'une quelconque des revendications 1 à 4, dans lequel l'orifice décalé (11c) du collecteur (18) est en communication de fluide avec une source de pression négative.

6. Système selon l'une quelconque des revendications 1 à 5, dans lequel l'un desdits orifices dudit collecteur dudit cathéter de pose à plusieurs lumières est en communication de fluide avec un réservoir de fluide de dissolution.

7. Ensemble destiné à être utilisé pour rincer un site vasculaire avec du fluide, ledit ensemble comprenant un système selon l'une quelconque des revendications précédentes et les instructions pour son utilisation.
